# EUROPEAN PATENT SPECIFICATION

(11) **EP 3 322 402 B1**
(45) Date of publication and mention of the grant of the patent: **29.05.2019**
(21) Application number: 16748275.1
(22) Date of filing: 15.07.2016
(51) Int. Cl.: A61K 9/00, A61K 31/5517, A61K 47/38, A61P 25/08

(54) **COMPOSITIONS OF MIDAZOLAM FOR BUCCAL ADMINISTRATION IN THE TREATMENT OF SEIZURES TO OBTAIN RAPID ONSET OF ACTION**
ZUSAMMENSETZUNGEN VON MIDAZOLAM ZUR BUKKALEN VERABREICHUNG BEI DER BEHANDLUNG VON ANFÄLLEN ZUM ERHALT EINES SCHNELLEN WIRKUNGSEINTRITTS
COMPOSITIONS DE MIDAZOLAM DESTINÉES À UNE ADMINISTRATION BUCCALE DANS LE TRAITEMENT DE CRISES AFIN D'OBTENIR UN DÉLAI D'ACTION RAPIDE

(30) Priority: 16.07.2015 DK 201570475
(43) Date of publication of application: 23.05.2018
(73) Proprietor: SWIPP AB, 223 61 Lund (SE)
(72) Inventor: SJÖGREN, Christer, 263 61 Viken (SE)
(74) Representative: Chas. Hude A/S
(86) International application number: PCT/EP2016/066860
(87) International publication number: WO 2017/009446

(56) References cited:
- WO-A1-99/09989
- WO-A1-2011/053251
- DATABASE STN CHEMICAL ABSTRACTS, X [Online] 15 August 1988 (1988-08-15), XP002055633, retrieved from CHEMICAL Database accession no. 47764

## Description

### Field of the invention

The present invention provides film compositions of midazolam for use in the treatment of seizures eg in connection with epilepsy or febrile seizures. The film-compositions are intended for administration to the oral cavity in the form of a bio-adhesive film. The composition makes it possible to administer the drug substance, midazolam, to a patient, even if the patient is unconscious and it avoids the risk that the patient receives a too low dose eg if a solution is administered, and some of the applied solution may flow out of the mouth instead of being absorbed by the oral mucosa.

### Background of the invention

Midazolam is a well-documented drug substance with sedative, anxiolytic, amnesic and hypnotic properties.

Midazolam acts on nerve cells in the brain and is used to control convulsions (fits or seizures) in children and adults with epilepsy or other diseases associated with seizures, or in children with febrile seizures. The brain and nerves are made up of many nerve cells that communicate with each other through electrical signals. These signals must be carefully regulated for the brain and nerves to function properly.

When abnormally rapid and repetitive electrical signals are evoked in the brain, it becomes over-stimulated and normal function is disturbed. This results in fits or seizures. Midazolam works by amplifying the action of the neurotransmitter GABA in the brain. GABA is involved in transmitting messages between the nerve cells and acts as a natural 'nerve-calming' agent. It helps keep the nerve activity in the brain in balance, and is involved in inducing sleepiness, reducing anxiety and relaxing muscles.

Midazolam is commercially available in the form of its hydrochloride, for example in the form of a glycerine-based syrup sold under the trade name VERSED®, which contains 2.5 mg/mL of midazolam. Midazolam is also marketed in the form of its maleate salt for example in tablets containing 7.5 or 15 mg per tablet under the trademark DOR-MICUM®. Products, which are formulated for administration via the buccal route, are EPISTATUS® (10 mg/mL midazolam in liquid form) and BUCCOLAM® (2.5mg/0.5 mL, 5 mg/mL, 7.5 mg/mL or 10 mg/2 mL midazolam in liquid form).

EP-B-1 323 422 (Special Products Limited) relates to a liquid midazolam composition, which contains a higher concentration of midazolam than prior art compositions. The composition contains midazolam maleate dissolved in an aqueous liquid medium containing ethanol and a polyhydroxy solvent such as glycerol or propylene glycol.

WO 99/09989 A1 discloses a pharmaceutical composition for oral administration comprising a carrier and, as active ingredient, midazolam or a salt thereof, characterized in that the composition is in the form of a fast-dispersing dosage form designed to release the active ingredient rapidly in the oral cavity.

Buccally administered midazolam given in recommended doses shows a rapid absorption. Furthermore, the achieved plasma levels are sufficient to provide therapeutic effect in the patient. However, in view of the importance of rapid delivery of midazolam to patients in need thereof, it would be advantageous if the delivery of the drug could be safer, more simplified and if a lower dose could be used. A lower dose leading to the desired therapeutic effect could reduce frequency and seriousness of side-effects such as eg respiratory depression.

### Detailed description of the invention

The present invention addresses the above-mentioned needs and provides a composition in the form of a film containing midazolam for application to the oral mucosa or tongue. The film in unit dose form contains from 0.25 to 2 mg midazolam either in the form of midazolam or a pharmaceutically acceptable salt thereof, and a film-forming substance. Notably, as it appears from the examples herein, buccal administration of a dose of 1 mg midazolam in the form of a film preparation according to the invention to an adult with a weight of 90 kg leads to a plasma level that is therapeutically effective (about 5 ng/mL). An important object of the invention is to provide a film preparation that is suitable for use in children suffering from seizures and the examples herein indicate that a film preparation according to the invention containing 1 mg or less such as 0.75 mg, 0.5 mg or 0.25 mg or even as low as 0.1 mg of midazolam will be suitable for administration to children to achieve the desired and fast therapeutic effect. The dose will depend on the weight of the child.

A low-dose midazolam composition, which at the same time is therapeutically effective, is expected to have substantial impact on the safe use of the drug. Known side-effects are respiratory depression, respiratory arrest, apnoea, hypotension, bradycardia, heart arrest, death. Moreover, an increased content in the blood of metabolites of midazolam increased the risk for respiratory depression, especially in children. To this end, it should be noted that in children the level of metabolites are higher than the level seen in adults, which especially increases the risks for toxic effects in children.

Surprisingly, the present inventor has found that buccal administration of midazolam via a film composition is very efficient and a dose as low as 2 mg leads to blood levels of 5-10 ng/mL in adult volunteers. Even a dose of 1 mg to adults with an average body weight of 90 kg results in therapeutic levels of midazolam. There is a linear relationship between dose and plasma level and therefore the dose to children could be as low as 0.011mg/kg body weight (corresponding to 0.22 mg for a child weighing 20 kg)

It has recently be shown in healthy volunteers that buccal administration of midazolam solution 5 mg gave rise to blood levels of 5-10 ng/mL within 3-6 min (Schwagmeier et al. 1998). In studies in patients, an anticonvulsive effect is frequently achieved within 5 minutes in children using a buccal dose of 0.2 mg/kg (Talukbar et al. 2009) and in adults administered 10-20 mg (Nakken et al. 2011). Scott et al. (1998) observed that in adult volunteers given approx. 10 mg midazolam a plasma concentration of 5 ng/mL was reached in most of the subjects in 5-10 minutes.

In general, it is contemplated that the plasma level of midazolam should be from about 5 to about 20 ng/mL to obtain the desired effect in the management of seizures. In the present context management of seizures include febrile seizures, acute seizure and status epilepticus. Epileptic seizures (convulsive status epilepticus) are a common cause of neurological medical emergency and often result in brain damage. Failure to relieve the symptoms of an epileptic fit in less than about 15 minutes can lead to death. Thus, it is extremely important to treat the patient promptly and to relieve the symptoms as quickly as possible in order to minimize the risk of brain damage of death of the patient.

In a composition of the invention, midazolam may be used in the form of midazolam or in the form of pharmaceutically acceptable salts thereof. Such salts include salts formed from inorganic or organic acids such as hydrochloride, sulfate, phosphate, maleate, fumarate and the like. In the event a salt is employed, the amount of midazolam in the film is given as the free base.

A film composition according to the invention contains a film-forming substance. Film-forming agents suitable for use include cellulose and cellulose derivatives such as ethylcellulose, methylcellulose, hydroxypropyl methylcellulose, hydroxyethyl cellulose, hydroxypropyl cellulose, carboxymethylcellulose (and its sodium salt), microcrystalline cellulose, crosscarmellose, cellulose acetate;
polysaccharides such as alginate (or alginic acid), pectin, tragacanth, carrageenan, dextrans, gelatin, sclerogucan, xanthan gum, guar gum: acrylic polymers or co-polymer;
polyvinylpyrrolidones, polyvinylalcohol;
polyethylene oxides;
or mixtures thereof.

As seen from the examples herein hydroxypropyl methylcellulose (HPMC) may be used as the film-forming agent in the present invention. Suitable grades of HPMC and possible other film-forming agents for use in an oral film according to the invention are described herein. The concentration of HPMC(s) in a film is normally in a range of from 35 to 99% w/w. The total concentration of HPMCs may be from about 40 to about 99% w/w, from about 45 to about 99% w/w or from about 50 to 99% w/w. As seen from the examples herein the range may be from 80 to 99% w/w, notably about 98% w/w. The % w/w are given based on total dry matter in the film.

In short, the HPMC for use in a film according to the invention may be as specified in Ph. Eur., it may be type 2910 and/or it may be a mixture of two or more HPMCs having different viscosities. In the latter case, the concentrations in the film of the two or more HPMCs having different viscosity may be the same or different, notably the same. As seen from the examples herein, where two HPMCs are present in an oral film the concentration of the first HPMC may be from 25-50 % w/w and the concentration of the second HPMC may be from 10-45% w/w and the sum concentration of the two HPMCs is from 35-99% w/w. In the case that more HPMCs are present in an oral film, the individual concentration may span over the range 10-50% w/w of each HPMC and the sum concentration of all HPMCs is from 35-99% w/w.

Moreover, in such situations where there is a mixture of two HPMCs these HPMCs may have a viscosity of 3 mPa s and 50 mPa s, respectively.

As it appears from the examples herein a film according to the present invention may contain a mixture of HPMCs having a viscosity of 3 mPa s and 50 mPa s, respectively. The total concentration of HPMCs may be as described above. In specific film preparations the individual concentrations of HPMC 3 mPa s and HPMC 50 mPa s are the same such as, eg, in a range of from 20% w/w to 50% w/w for each HPMC and based on total dry matter in the oral film. As seen from the examples herein films with suitable properties are obtained for a film according to the invention that contains a mixture of HPMCs having a viscosity of 3 mPa s and 50 mPa s, respectively, and a total concentration of HPMC s of from 80 to 99% w/w (based on total dry matter).

Many of the substances mentioned above are obtained with different properties, different molecular weight etc. Thus, polyethylene oxide can be obtained with average molecular weights from about 20,000 to about 4,000,000 Daltons, and acrylic polymers can be purchased e.g. with different solubilities in different media. Thus, in the event that eg a faster release is desired the content of film-forming polymer may be varied to adjust the release rate. Most of the above-mentioned film-forming substances are also bio-adhesive to a varying degree.

Apart from midazolam and a film-forming substance, a composition of the invention may also contain one or more pharmaceutically acceptable excipients or additives including solubilizers, pH adjusting agents, stabilizers, taste-masking agent(s), colouring agents, anti-oxidants (if needed), etc.

A film or wafer is typically a paper-thin polymer film that is used as carriers for drug substance(s). Normally, a film composition is thicker than a piece of paper, but normally the thickness is 50 - 500 micrometer. A wafer as well as a film is administered orally and does not require water or swallowing.

The film typically contain 0.5-20 mg midazolam/g of the film composition. The composition is typically in the form of a unit dose composition and generally, the composition has a weight of from about 50 to about 200 mg. In general the dose is from about 0.01 to 0.2 mg/body weight, but as seen from the examples herein, the dose may be reduced to from about 0.01 -0.1 mg/kg body weight, notably from about 0.01 to 0.02 mg/kg body weight or that each unit dose will contain approximately a dose of 0.1 - 2 mg or from 0.1 to 1.5 mg or from 0.1 to 1.0 mg for children and adults weighing 10-90 kg.

A composition of the invention is typically prepared by dissolving midazolam in a suitable solvent such as a water or ethanol or a water-ethanol mixture. The film-forming agent is dissolved either in the same medium or in a solvent or solvent mixture, wherein the film-forming substance is soluble. Water may be included, but should normally not exceed about 50% v/v of the solvent in order to ensure a suitable, not too long drying time. The solutions containing midazolam and the film-forming substance are mixed, film-casted and dried. If further excipients/additions are desired in the composition these substances are added to one of the solutions. The midazolam and the film-forming substance may also be dissolved in one pot. In some cases, midazolam may be employed in micronized form; this is especially the case if midazolam is not dissolved or only partly dissolved in the solvent.

After application, either on the tongue or the oral mucosa, the desired plasma level will be achieved in 3-5 minutes. Due to the low doses and the fact that the composition does not flow out of the mouth, it is also possible to repeat the dosing, if necessary, already after 10-15 minutes without any risk of side-effects as prolonged drowsiness, respiratory depression etc.

In other aspects of the invention, the film preparations may be used in the treatment of seizures in cats or dogs. The dose is believed to be of the same order of magnitude. The following examples are for illustrative purposes only and are not intended to limit the scope of the invention.

### Example 1

### A film composition of the invention

| | **%w/w** |
|---|---|
| Hydroxypropylmethylcellulose | 99.8 |
| Midazolam | 0.2 |

HPMC and midazolam is dissolved/dispersed in ethanol-water (5:1) mixture and casted. The composition is dried at 40 °C.

Ethanol-water mixtures can be used, wherein the concentration of ethanol in the final mixture is from 50-100% v/v.

### Examples 2-10

Compositions as described in Example 1 are prepared using:
Example 2: hydroxypropylcellulose instead of HPMC
Example 3: ethylcellulose instead of HPMC
Example 4: carboxymethylcellulose instead of HPMC
Example 5: Polyvinylpyrrolidone instead of HPMC
Example 6: Cellulose acetate instead of HPMC
Example 7: Polyvinyl alcohol instead of HPMC
Example 8: Alginate instead of HPMC
Example 9: Dextran 400,000 instead of HPMC
Example 10: Polyethylene oxide 700,000 instead of HPMC

### Examples 11-19

Examples 2-10 are repeated, but using a 1:1 mixture of the stated polymer and HPMC.

### Example 20

### A film composition of the invention

| | |
|---|---|
| Midazolam hydrochloride | 60 mg |
| Hypromellose E3 | 2.5 g |

| | |
|---|---|
| Hypromellose E50 | 2.5 g |

The above-mentioned ingredients are mixed in 30 ml of ethanol overnight. Then the gel is casted on a glass plate and dried at 40°C for 30 minutes. 1 g of the film contains 1.2 mg of midazolam hydrochloride.

### Example 21

### Pilot in vivo study

Film preparations weighing approximately 0,07 g (1 x 2 cm) containing 2 mg midazolam hydrochloride were applied on the oro-mucosal membrane in healthy volunteers (body weight 90 kg). Venous blood samples were drawn 6, 12 and 18 minutes after application. Plasma levels ranging from 3 to 10 ng/mL (measured by LC-MS/MS after liquid extraction) were achieved in the volunteers. The achieved plasma levels in adults will correspond to at least 15-50 ng/mL in children weighing 20 kg. No side-effects were observed. Average plasma levels of 5 ng/mL were obtained as described herein.

### Example 22

### Pilot in vivo study

Film preparations weighing approximately 0,07 g (1 x 2 cm) containing 1 mg midazolam hydrochloride were applied on the oro-mucosal membrane in healthy volunteers (body weight 90 kg). Venous blood samples were drawn 6, 12 and 18 minutes after application. Plasma levels ranging from 1 ng/mL (6 min) to 4.6 ng/mL (12 min) (measured by LC-MS/MS after liquid extraction) were achieved in the volunteers. The achieved plasma levels in adults will correspond to at least 15-20 ng/mL in children weighing 20 kg. No side-effects were observed. Average plasma levels were obtained as described herein.

The film preparation used were based on midazolam and HPMC (only one quality of HPMC); however, later studies have shown that the use of the combination of HPMC as described in example 20 gives improved results due to the structure and solubility of the film. Thus, it is expected that even higher concentrations midazolam can be achieved using a formulation containing a combination of Hypromellose E3 and Hypromellose E50, notably in a weight ratio of from 1:5 to 5:1 such as 1:1.

## Claims

1. A unit dosage composition in the form of a film containing from 0.25 mg to 2 mg of midazolam or a pharmaceutically acceptable salt thereof, and a film-forming substance.

2. A unit dosage composition according to claim 1 containing from 0.01 to 1 mg of midazolam or a pharmaceutically acceptable salt thereof, and a film-forming substance.

3. A unit dosage composition according to claim 1 or 2 containing from 0.01 to 0.75 mg of midazolam or a pharmaceutically acceptable salt thereof, and a film-forming substance.

4. A unit dosage composition according to any of the preceding claims containing from 0.01 to 0.5 mg of midazolam or a pharmaceutically acceptable salt thereof, and a film-forming substance.

5. A unit-dosage composition according to any of the preceding claims, wherein the film-forming substance is selected from cellulose and cellulose derivatives such as ethylcellulose, methylcellulose, hydroxypropyl methylcellulose, hydroxyethyl cellulose, hydroxypropyl cellulose, carboxymethylcellulose (and its sodium salt), microcrystalline cellulose, crosscarmellose, cellulose acetate, or mixtures thereof.

6. A unit dosage composition according to any one of claims 1-4, wherein the film-forming substance is a polysaccharide selected from the group consisting of alginate (or alginic acid), pectin, tragacanth, carrageenan, dextrans, gelatin, sclerogucan, xanthan gum, guar gum, and mixtures thereof.

7. A unit dosage composition according to any one of claims 1-4, wherein the film-forming substance is an acrylic polymer or co-polymer, or mixtures thereof.

8. A unit dosage composition according to any one of claims 1-4, wherein the film-forming substance is polyvinylpyrrolidone or polyvinylalcohol, or mixtures thereof.

9. A unit dosage composition according to any one of claims 1-4, wherein the film-forming substance is a polyethylene oxide.

10. A unit dosage composition according to any one of claims 1-5, wherein the film-forming substance is a mixture of hydroxypropyl methylcelluloses.

11. A unit dosage form according to any of the preceding claims for use in the treatment of seizures including epileptic seizures and status epilepticus.

12. A unit dosage form according to any of the preceding claims for application to the oral cavity of a human.

13. A unit dosage form as defined in any one of claims 1 -12 for use in the treatment of epileptic seizures, other forms of seizures, or status epilepticus of a human, wherein the unit dosage form is administered to the oral cavity of the human in need thereof.

14. A unit dosage form for use according to claim 13, wherein a therapeutically effective concentration of midazolam in the blood of said human is obtained within 3-5 minutes after application to the oral cavity.

15. A unit dosage form for use according to claim 13 or 14, wherein a therapeutically effective concentration of midazolam in the blood of said human is in a range of from 5 to 20 ng/mL or higher.

## Patentansprüche

1. Eine Dosierungseinheitszusammensetzung in der Form eines Films, die zwischen 0,25 mg bis 2 mg an Midazolam oder einem pharmazeutisch annehmbaren Salz davon enthält, und eine filmbildende Substanz.

2. Eine Dosierungseinheitszusammensetzung nach Anspruch 1, die zwischen 0,01 bis 1 mg von Midazolam oder einem pharmazeutisch annehmbaren Salz davon enthält, und eine filmbildende Substanz.

3. Eine Dosierungseinheitszusammensetzung nach Anspruch 1 oder 2, die zwischen 0,01 bis 0,75 mg von Midazolam oder einem pharmazeutisch annehmbaren Salz davon enthält, und eine filmbildende Substanz.

4. Eine Dosierungseinheitszusammensetzung nach einem der vorhergehenden Ansprüche, die zwischen 0,01 bis 0,5 mg von Midazolam oder einem pharmazeutisch annehmbaren Salz davon enthält, und eine filmbildende Substanz.

5. Eine Dosierungseinheitszusammensetzung nach einem der vorhergehenden Ansprüche, wobei die filmbildende Substanz ausgewählt ist aus Cellulose und Cellulosederivaten, wie Ethylcellulose, Methylcellulose, Hydroxypropylmethylcellulose, Hydroxyethylcellulose, Hydroxypropylcellulose, Carboxymethylcellulose (und ihr Natriumsalz), Mikrokristallincellulose, Crosscramelose, Celluloseacetat oder Mischungen davon.

6. Eine Dosierungseinheitszusammensetzung nach einem der Ansprüche 1-4, wobei die filmbildende Substanz ein Polysaccharid ist, ausgewählt aus der Gruppe bestehend aus Alginat (oder Alginsäure), Pektin, Tragacanth, Karrageen, Dextrane, Gelatin, Sclerogucan, Xanthan, Guaran und Mischungen davon.

7. Eine Dosierungseinheitszusammensetzung nach einem der Ansprüche 1-4, wobei die filmbildende Substanz ein akrylisches Polymer oder Co-Polymer ist oder Mischungen davon.

8. Eine Dosierungseinheitszusammensetzung nach einem der Ansprüche 1-4, wobei die filmbildende Substanz Polyvinylpyrrolidon oder Polyvinylalkohol ist oder Mischungen davon.

9. Eine Dosierungseinheitszusammensetzung nach einem der Ansprüche 1-4, wobei die filmbildende Substanz ein Polyethylenoxid ist.

10. Eine Dosierungseinheitszusammensetzung nach einem der Ansprüche 1-5, wobei die filmbildende Substanz eine Mischung von Hydroxypropylmethylcellulose ist.

11. Eine Dosierungseinheitsform nach einem der vorhergehenden Ansprüche zur Verwendung bei der Behandlung von Krämpfen, einschließlich epileptischer Anfälle und Status epilepticus.

12. Eine Dosierungseinheitsform nach einem der vorhergehenden Ansprüche zur Verabreichung in die Mundhöhle eines Menschen.

13. Eine Dosierungseinheitsform wie in einem der Ansprüche 1-12 definiert zur Verwendung bei der Behandlung von epileptischen Anfällen, anderen Arten von Krämpfen oder Status epilepticus eines Menschen, wobei die Dosierungseinheitsform in die Mundhöhle eines Menschen, der daran Bedarf hat, verabreicht wird.

14. Eine Dosierungseinheitsform zur Verwendung nach Anspruch 13, wobei die therapeutisch effektive Konzentration an Midazolam im Blut jenes Menschen innerhalb von 3-5 Minuten nach der Verabreichung in die Mundhöhle erreicht wird.

15. Eine Dosierungseinheitsform zur Verwendung nach Anspruch 13 oder 14, wobei eine therapeutisch effektive Konzentration von Midazolam im Blut jenes Menschen im Bereich von 5 bis 20 ng/ml oder höher ist.

## Revendications

1. Composition à dose unitaire sous la forme d'un film contenant de 0,25 mg à 2 mg de midazolam ou d'un sel pharmaceutiquement acceptable de celui-ci, et une substance filmogène.

2. Composition à dose unitaire selon la revendication 1 contenant de 0,01 à 1 mg de midazolam ou d'un sel pharmaceutiquement acceptable de celui-ci, et une substance filmogène.

3. Composition à dose unitaire selon la revendication 1 ou 2 contenant de 0,01 à 0,75 mg de midazolam ou d'un sel pharmaceutiquement acceptable de celui-ci, et une substance filmogène.

4. Composition à dose unitaire selon l'une quelconque des revendications précédentes contenant de 0,01 à 0,5 mg de midazolam ou d'un sel pharmaceutiquement acceptable de celui-ci, et une substance filmogène.

5. Composition à dose unitaire selon l'une quelconque des revendications précédentes, dans laquelle la substance filmogène est choisie parmi la cellulose et les dérivés de la cellulose tels que l'éthylcellulose, la méthylcellulose, l'hydroxypropylméthylcellulose, l'hydroxyéthylcellulose, l'hydroxypropylcellulose, la carboxyméthylcellulose (et son sel de sodium), la cellulose microcristalline, la crosscarmellose, l'acétate de cellulose, ou les mélanges de ceux-ci.

6. Composition à dose unitaire selon l'une quelconque des revendications 1 à 4, dans laquelle la substance filmogène est un polysaccharide choisi dans le groupe constitué de l'alginate (ou acide alginique), de la pectine, de la gomme adragante, de la carraghénine, des dextranes, de la gélatine, du sclérogucane, de la gomme xanthique, de la gomme de guar, et des mélanges de ceux-ci.

7. Composition à dose unitaire selon l'une quelconque des revendications 1 à 4, dans laquelle la substance filmogène est un polymère ou copolymère acrylique, ou des mélanges de ceux-ci.

8. Composition à dose unitaire selon l'une quelconque des revendications 1 à 4, dans laquelle la substance filmogène est de la polyvinylpyrrolidone ou de l'alcool polyvinylique, ou des mélanges de ceux-ci.

9. Composition à dose unitaire selon l'une quelconque des revendications 1 à 4, dans laquelle la substance filmogène est un oxyde de polyéthylène.

10. Composition à dose unitaire selon l'une quelconque des revendications 1 à 5, dans laquelle la substance filmogène est un mélange d'hydroxypropylméthylcelluloses.

11. Forme à dose unitaire selon l'une quelconque des revendications précédentes pour utilisation dans le traitement de crises incluant les crises épileptiques et l'état de mal épileptique.

12. Forme à dose unitaire selon l'une quelconque des revendications précédentes pour application à la cavité orale d'un humain.

13. Forme à dose unitaire selon l'une quelconque des revendications 1 à 12 pour utilisation dans le traitement de crises épileptiques, d'autres formes de crises, ou de l'état de mal épileptique d'un humain, où la forme à dose unitaire est administrée à la cavité orale de l'humain qui le nécessite.

14. Forme à dose unitaire selon la revendication 13, où une concentration thérapeutiquement efficace de midazolam dans le sang dudit humain est obtenue dans les 3 à 5 minutes après application à la cavité orale.

15. Forme à dose unitaire selon la revendication 13 ou 14, où une concentration thérapeutiquement efficace de midazolam dans le sang dudit humain est dans une plage de 5 à 20 ng/ml ou supérieure.
